(19) Europäisches Patentamt
European Patent Office
Office européen des brevets

(11) Publication number: **0 007 404**
**A1**

(12) # EUROPEAN PATENT APPLICATION

(21) Application number: 79101780.9

(22) Date of filing: 05.06.79

(51) Int. Cl.³: C 07 C 35/36
C 07 C 49/577, C 07 C 49/755
C 07 C 45/30, C 07 C 45/00
C 07 C 43/23

(30) Priority: 05.06.78 US 912841

(43) Date of publication of application:
06.02.80 Bulletin 80/3

(84) Designated Contracting States:
AT BE CH DE FR GB IT NL SE

(71) Applicant: KANSAS UNIVERSITY ENDOWMENT ASSOCIATION
226 Strong Hall
Lawrence, Kansas(US)

(72) Inventor: Alexander, Jose
4117 Adams
Kansas City, Kansas(US)

(72) Inventor: Mitscher, Lester Allen
1903 Kasold
Lawrence, Kansas(US)

(74) Representative: Körner, Ekkehard, Dipl.-Ing. et al,
Patentanwälte Müller-Börner, Wey & Körner
Widenmayerstrasse 49
D-8000 München 22(DE)

(54) **Synthesis of 2-hydroxy-2-acetyl-1,2,3,4-tetrahydronaphthalene compounds.**

(57) A process for synthesizing 5,8-dialkoxy-2- hydroxy-2-acetyl- 1,2,3,4-tetrahydronaphthalene and 5,8-dibenzyloxy-2-hydroxy-2-acetyl- 1,2,3,4-tetrahydronaphthalene. These compounds are useful as intermediates in the synthesis of doxorubicin, daunomycin and their analogs.

EP 0 007 404 A1

TITLE MODIFIED
see front page

## Tetrahydronaphthalene Synthesis

This invention relates to techniques for synthesizing precursors of doxorubicin and related compounds such as daunomycin and carminomycin, and the aglycones thereof. More specifically, the present invention pertains to a technique for synthesizing 5,8-dialkoxy-2-hydroxy-2-acetyl-1,2,3,4-tetrahydronaphthalene and 5,8-dibenzyloxy-2-hydroxy-2-acetyl-1,2,3,4-tetrahydronaphthalene, which compounds may be converted into anthracycline antibiotics, such as daunomycin and doxorubicin, using established techniques.

Doxorubicin is a known anthracycline antibiotic described, e.g., in U.S. Patent No. 3,590,028. Doxorubicin, and the closely related compound daunomycin, are antineoplastic agents of established clinical utility. Doxorubicin hydrochloride, available from Adria Laboratories, Inc., under the trade name Adriamycin®, has been approved by the United States Food and Drug Administration for use in clinical research, and is one of the most powerful anticancer drugs available against numerous forms of cancer.

At present, doxorubicin is produced commercially from a soil fungus by a fermentation process. Such techniques are inherently expensive and limit the types of molecules that can be produced. Because of the inherent disadvantages of presently available commercial techniques for producing doxorubicin and related compounds, substantial effort has been devoted to developing processes for producing such compounds by chemical synthesis.

Techniques for the synthesis of anthracycline antibiotics such as doxorubicin and daunomycin are known. See, e.g., Wong et al, Canadian Journal of Chemistry, Vol. 51, page 466 (1973); Acton et al, Journal of Medicinal Chemistry, Vol. 17, No. 6, page 659 (1974); Kende et al,

Journal of the American Chemical Society, Vol. 97, No. 15, page 4425 (1975) and Vol. 98, No. 7, page 1967 (1976); Sih et al, Tetrahedron Letters, page 3385 (1976); Swenton et al, Tetrahedron Letters, page 2383 (1977); and Kelly et al, Journal of the American Chemical Society, Vol. 99, page 5513 (1977). None of the known techniques for the total synthesis of anthracycline antibiotics such as daunomycin and doxorubicin have yet been proven to be commercially successful. Because of the demand for, and scarcity of, these compounds, a suitable synthesis technique is highly desired.

The present invention provides a suitable synthesis technique by providing precursors for synthesizing daunomycin and related compounds, which may be prepared from readily available and inexpensive starting materials. In accordance with the present invention, 5,8-dialkoxy-2-hydroxy-1,2,3,4-tetrahydronaphthalene or 5,8-dibenzyloxy-2-hydroxy-1,2,3,4-tetrahydronaphthalene are oxidized to produce 5,8-dialkoxy-2-keto-1,2,3,4-tetrahydronaphthalene or 5,8-dibenzyloxy-2-keto-1,2,3,4-tetrahydronaphthalene. These latter compounds are reacted with the lithium salt of methylvinyl ether to produce the desired 5,8-dialkoxy-2-hydroxy-2-acetyl-1,2,3,4-tetrahydronaphthalene or 5,8-dibenzyloxy-2-hydroxy-2-acetyl-1,2,3,4-tetrahydronaphthalene.

The sequence of reactions described above provides a novel and convenient method for the preparation of 5,8-dialkoxy- or 5,8-dibenzyloxy-2-hydroxy-2-acetyl-1,2,3,4-tetrahydronaphthalene, which compounds are useful intermediates in the synthesis of doxorubicin, daunomycin, carminomycin, 4-demethoxydaunomycin and related antitumor agents and their aglycones in accordance with established techniques such as those disclosed in, e.g., C. M. Wong et al, Canadian Journal of Chemistry, Vol. 49, page 2712 (1971); Vol. 51, page 466 (1973); and F. Arcamone et al, Cancer Treatment Reports, Vol. 60, page 829 (1976).

In the synthesis of the present invention, 5,8-alkoxy- or 5,8-benzyloxy-2-hydroxy-1,2,3,4-tetrahydronaphthalene is used as a starting material. These compounds may be prepared by allowing commercially available and inexpensive

butadiene and p-benzoquinone to react under the normal conditions of the Diels-Alder reaction [See O. Diels and K. Alder, Ber., 62, page 2337 (1929)] to give the well-known adduct 1,4,4a,5,8,8a-cis-hexahydro-5,8-dioxonaphthalene of the following formula:

1.

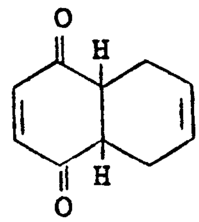

Alkylation, with a $C_1$-$C_6$ alkyl group, or benzylation under alkaline conditions produces the non-conjugated dihydronaphthalene derivates of the following formula:

2.

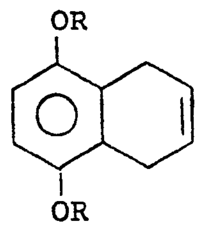

in which R represents an alkyl group of from 1 to 6 carbon atoms or a benzyl moiety. These compounds can be referred to as 5,8-dialkoxy-1,4-dihydronaphthalene or 5,8-dibenzyloxy-1,4-dihydronaphthalene.

Hydration of the double bond, as for example under hydroboration-oxidation conditions, produces the desired tetrahydronaphthol compounds used as starting materials in the process of the present invention. The 5,8-dialkoxy-2-hydroxy-1,2,3,4-tetrahydronaphthalene or 5,8-dibenzyloxy-2-hydroxy-1,2,3,4-tetrahydronaphthalene compounds have the formula:

3.

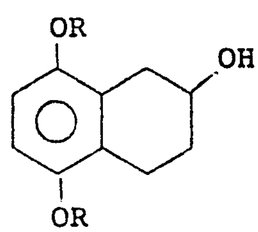

in which R represents an alkyl group of from 1 to 6 carbon atoms or a benzyl moiety.

The compounds of formula 3 are oxidized, preferably with pyridinium chlorochromate, to produce 5,8-alkoxy-2-keto-1,2,3,4-tetrahydronaphthalene or 5,8-dibenzyloxy-2-keto-1,2,3,4-tetrahydronaphthalene having the formula:

4. 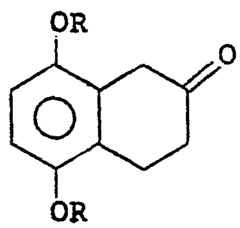

where R is as previously defined. The compound of formula 4 where R is $CH_3$ is known. [See T. R. Lewis et al, Journal of the American Chemical Society, Vol. 74, page 5321 (1952) and G. Stork et al, ibid., Vol. 94, page 4735 (1972)].

The method described above of preparing the ketones of formula 4 is more efficient than the methods recorded in the literature [e.g., A. P. Tarentev and P. P. Shavolova, J. Gen. Chem. U.S.S.R., Vol. 15, page 142 (1945); T. R. Lewis, W. B. Dickinson and S. Archer, Journal of the American Chemical Society, Vol. 74, page 5321 (1952)].

Reaction of the ketone of formula 4 with the lithium salt of methylvinyl ether, followed by acidic work-up, produces the desired 5,8-dialkoxy- or 5,8-dibenzyloxy-2-hydroxy-2-acetyl-1,2,3,4-tetrahydronaphthalene compounds having the formula

5. 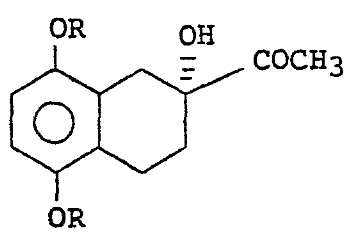

in which R is as previously defined. The compounds of formula 5 where R is $CH_3$ are also known; see C. M. Wong et al, Canadian Journal of Chemistry, Vol. 49, page 2712 (1971) and Vol. 51, page 466 (1973).

The compounds of formula 5 may be converted to anthracycline antibiotics, such as doxorubicin, daunomycin, carminomycin, and 4-demethoxydaunomycin, and their aglycones by known techniques. For example, C. M. Wong et al,

Canadian Journal of Chemistry, Vol. 51, page 466 (1973), discloses a technique for synthesizing daunomycinone, the aglycone of daunomycin, from 5,8-dimethyl-2-hydroxy-2-acetyl-1,2,3,4-tetrahydronaphthalene (the compound of formula 5, where R is $CH_3$). While the technique disclosed in Wong et al specifically employs the 5,8-dimethyl compound, any of the other compounds of formula 5 (i.e., where R is benzyl or $C_2$-$C_6$ alkyl) can be used in place of the 5,8-dimethyl compound for producing daunomycinone by the Wong et al technique.

Aglycones, such as daunomycinone, may be converted to the corresponding anthracycline antibiotic, e.g., daunomycin, by attaching daunosamine to the aglycone. Techniques for attaching daunosamine to the aglycones are disclosed in Acton et al, Journal of Medicinal Chemistry, Vol. 17, No. 6; page 659 (1974), and Smith et al, Journal of the American Chemical Society, Vol. 98, No. 7, page 1969 (1976).

The following examples further illustrate preferred embodiments of the present invention. The examples should in no way be considered limiting, but are merely illustrative of the various features of the present invention.

Example 1

5,8-Dimethoxy-2-hydroxy-1,2,3,4-tetrahydronaphthalene (2.4 g.) was dissolved in methylene chloride (20 ml.) and added in one portion to a suspension of pyridinium chlorochromate (5.2 g.) in methylene chloride (20 ml.). After stirring at room temperature for 3 hours, the reaction mixture was diluted with dry ether (100 ml.) and stirred until the sticky precipitate was transformed into a crisp solid (about 15 min.). The supernatant was decanted and the residue was leached with dry ether twice (25 ml. each). The combined ether solution was passed through a column of florisil (10 g.). The eluate on evaloperation furnished pure 5,8-dimethoxy-2-keto-1,2,3,4-tetrahydronaphthalene 1.6 g. (67.5%).

A very pure sample obtained by crystallization from ethanol melted at 98-99.5$^{O}$C. T. R. Lewis et al, Journal of the American Chemical Society, Vol. 74, page 5321 (1952)

reported a melting point of 98.6-99.4°C. See also G. Stork et al, Journal of the American Chemical Society, Vol. 94, page 4735 (1972).

The nuclear magnetic resonance spectrum (NMR) of the product exhibited the following peaks: ($CDCl_3$) δ2.48 (2H, t, J = 7 Hz, $CH_2C\underline{H}_2CO$), 3.06 (2H, t, J = 7 Hz, $ArC\underline{H}_2CH_2$), 3.48 (2H, s, $ArC\underline{H}_2CO$), 3.78 (6H, s, $OCH_3$), and 6.68 (2H, s, aromatic H). The infra red spectrum (IR) exhibited peaks at (nujol) 2942, 2840, 1712, 1648, 1600, 1478, 1250, 1075 $cm^{-1}$.

Example 2

To a cooled (-70°C.) solution of methylvinyl ether (≈2 g.) in dry tetrahydrofuran (THF) (20 ml.) was added dropwise a hexane solution of t-butyllithium (10 ml. of 1.6M solution) with stirring under argon. The yellow solution on warming up to -5°C. during 1/2 hr. became colorless. This solution of methoxyvinyllithium was then cooled to -70°C. and a solution of 5,8-dimethoxy-2-keto-1,2,3,4-tetrahydronaphthalene (0.5 g.) in dry THF (25 ml.) was added at such a rate that the temperature of the reaction mixture did not exceed -55°C.

The solution was allowed to warm up to 0°C. during 1 hr. After the addition of 0.5 ml. acetic acid, most of the solvents were evaporated from the reaction mixture. The residue was diluted with water and extracted with ether. The ether extract was washed 3 times with water, dired over $Na_2SO_4$ and evaporated. The residue, which contained unreacted starting material and the corresponding alcohol in addition to the desired product, was purified by chromatography to yield 114 mg. of 5,8-dimethoxy-2-hydroxy-2-acetyl-1,2,3,4-tetrahydronaphthalene.

The purified product had the following characteristics: NMR ($CDCl_3$) δ1.80 (broad triplet, 2H, alicyclic), 2.25 (3H, s, acetyl), 2.80 (4H, broad triplet, benzylic), 3.70 and 3.73 (3H each, s, $OC\underline{H}_3$), 3.56 (1H, s, OH, exchanged with $D_2O$) and 6.40 (2H, s, aromatic).

1. A process for producing 5,8-dialkoxy-2-hydroxy-2-acetyl-1,2,3,4-tetrahydronaphthalene or 5,8-dibenzyloxy-2-hydroxy-2-acetyl-1,2,3,4-tetrahydronaphthalene having the formula:

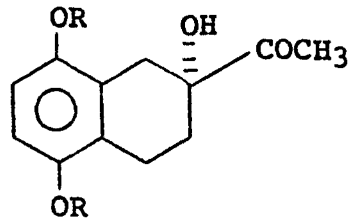

where R is a $C_1$ to $C_6$ alkyl or benzyl group, comprising:

a. oxidizing 5,8-dialkoxy-2-hydroxy-1,2,3,4-tetrahydronaphthalene or 5,8-dibenzyloxy-2-hydroxy-1,2,3,4-tetrahydronaphthalene having the formula

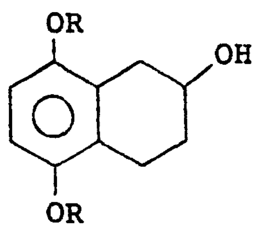

where R is a $C_1$ to $C_6$ alkyl or benzyl group to produce 5,8-dialkoxy-2-keto-1,2,3,4-tetrahydronaphthalene or 5,8-dibenzyloxy-2-keto-1,2,3,4-tetrahydronaphthalene having the formula

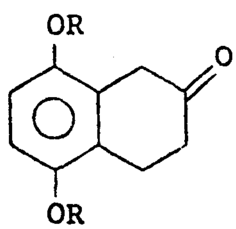

where R is a $C_1$ to $C_6$ alkyl or benzyl group, and

b. reacting said 5,8-dialkoxy-2-keto-1,2,3,4-tetrahydronaphthalene or 5,8-dibenzyloxy-2-keto-1,2,3,4-tetrahydronaphthalene with the lithium salt of methylvinyl ether to produce said 5,8-dialkoxy-2-hydroxy-2-acetyl-1,2,3,4-tetrahydronaphthalene or 5,8-dibenzyloxy-2-hydroxy-2-acetyl-1,2,3,4-tetrahydronaphthalene.

2. The process of claim 1 where R is a methyl group.

3. The process of claim 1 where acetic acid is added in step b. after reaction with said lithium salt of methylvinyl ether.

4. The process in accordance with any of claims 1, 2 or 3 wherein pyridinium chlorochromate is employed to achieve oxidation in step a.

5. A process which comprises reacting a tetrahydronaphthalene having the formula

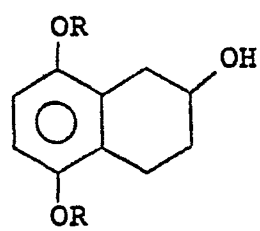

with pyridinium chlorochromate whereby said tetrahydronaphthalene is oxidized to produce a ketone having the formula

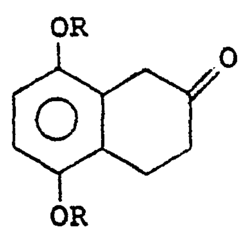

where R in both of the foregoing formulas is a $C_1$ to $C_6$ alkyl or a benzyl group.

6. A process which comprises

a. forming a reaction mixture containing as reactants the lithium salt of methylvinyl ether and a ketone having the formula

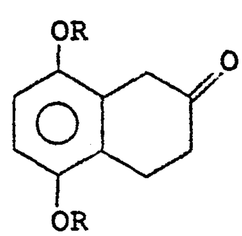

and

b. recovering from said reaction mixture a tetrahydronaphthalene having the formula

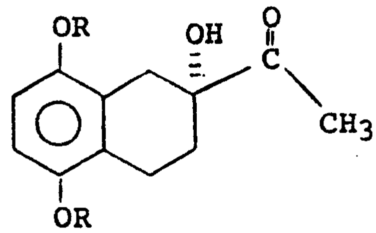

where R in both of the foregoing formulas is a $C_1$ to $C_6$ alkyl or benzyl group.

7. The process of claims 5 or 6 where R is a methyl group.

European Patent Office

# EUROPEAN SEARCH REPORT

Application number

EP 79 101 780.9

| Category | Citation of document with indication, where appropriate, of relevant passages | Relevant to claim |
|---|---|---|
| A | DE - A - 2 727 341 (SOCIETA FARMACEUTICI ITALIA)<br>* claim 1; position c *<br>-- | 1,2 |
| | Chemical Abstracts, Volume 83, Nr. 17, 1975<br>(COLUMBUS, OHIO, USA)<br>E.J. COREY et al. "Pyridinium chlorochromate. Efficient reagent for oxidation of primary and secondary alcohols to carbonyl compounds"<br>page 464, column 2, abstract Nr. 147058<br>& Tetrahedron Letters, Vol. 1975, Nr. 31, pages 2647 to 2650<br>---- | 4,5 |

CLASSIFICATION OF THE APPLICATION (Int. Cl3)

C 07 C 35/36
C 07 C 49/577
C 07 C 49/755
C 07 C 45/30
C 07 C 45/00
C 07 C 43/23

TECHNICAL FIELDS SEARCHED (Int.Cl.3)

C 07 C 35/36
C 07 C 43/22
C 07 C 49/43

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 28-08-1979 | KNAACK |